(19) Europäisches Patentamt
European Patent Office
Office européen des brevets

(11) EP 0 845 265 A1

(12) **EUROPEAN PATENT APPLICATION**
published in accordance with Art. 158(3) EPC

(43) Date of publication:
**03.06.1998 Bulletin 1998/23**

(21) Application number: **96926626.1**

(22) Date of filing: **12.08.1996**

(51) Int. Cl.$^6$: **A61K 38/00**, A61K 9/00

(86) International application number:
**PCT/JP96/02277**

(87) International publication number:
**WO 97/06813 (27.02.1997 Gazette 1997/10)**

(84) Designated Contracting States:
**AT BE CH DE DK ES FI FR GB GR IE IT LI LU MC NL PT SE**

(30) Priority: **15.08.1995 JP 208010/95**

(71) Applicants:
- **Asahi Kasei Kogyo Kabushiki Kaisha**
**Osaka-shi, Osaka 530-8205 (JP)**
- **HISAMITSU SEIYAKU KABUSHIKI KAISHA**
**Tosu-shi, Saga 841 (JP)**

(72) Inventors:
- **YAMAMOTO, Nakayuki**
**Shizuoka 419-01 (JP)**
- **ITO, Teruomi**
**Shizuoka 419-01 (JP)**

(74) Representative:
**Boeters, Hans Dietrich, Dr. et al**
**Patentanwälte Boeters & Bauer,**
**Bereiteranger 15**
**81541 München (DE)**

(54) **MUCOSAL PREPARATION CONTAINING PHYSIOLOGICALLY ACTIVE PEPTIDE**

(57) A preparation for transmucosal administration admixed with at least absorption promotor and vasodilator in physiologically active peptide is provided. Absorption of the physiologically active peptide can be increased by combining the peptide with absorption promotor and a compound having vasodilating activity. Accordingly auto-administration can be performed by the patients without pain of injection. It is useful for long term administration of physiologically active peptide.

FIG. 1

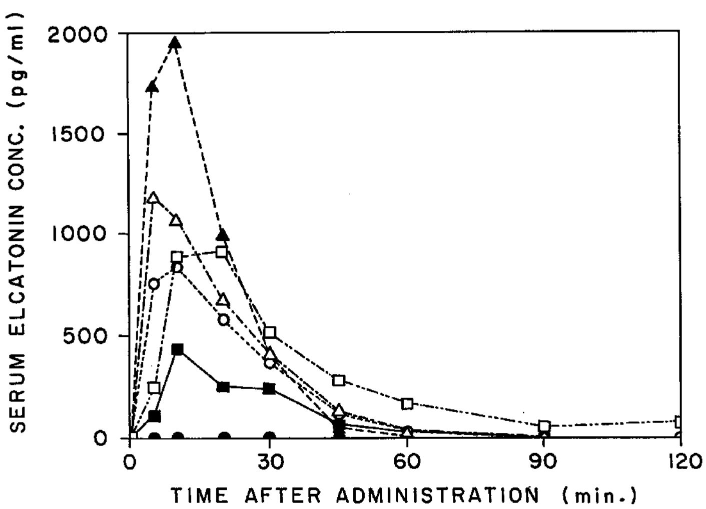


EP 0 845 265 A1

## Description

### FIELD OF THE INVENTION

The present invention relates to a preparation of peptide for transmucosal administration containing, at least, absorption promotor having absorption promoting action for the physiologically active peptide on nasal mucosa or rectal mucosa and a compound having vasodilative action. More particularly, the present invention relates to such the preparation of peptide for transmucosal administration having useful effects on medical care by absorbing the physiologically active peptide effectively through mucosa.

### PRIOR ARTS

Physiologically active peptide such as insulin and calcitonin are conventionally administered in the form of injections. However. administration by the injections requires patients on ambulaltory care with pain, and a preparation which can be administered by self-medication is desired.

Oral administration of the physiologically active peptides. which are poorly absorbed from digestive tract, involves decomposition of peptide by proteinase and first-pass effect in the liver, and has been of no practical use.

Recently, there have been many attempts to solve these problems for increasing absorption through nasal mucosa or rectal mucosa using preparations for transmucosal administration with various absorption promotors.

Nasal administration preparation with an absorption promotor has been tried, and such nasal administration preparations utilizing a salts of cholic acid having surface active agent action, for example sodium taurocholic acid, sodium cholic acid, sodium deoxycholic acid, sodium chenodeoxycholic acid, lysine chenodeoxycholic acid, sodium glycocholic acid, sodium glycodeoxycholic acid and lysine taurocholic acid (Japanese Patent Unexamined Publication No. 59-130820, EP-A-115,627, Japanese Patent Unexamined Publication No. 1-228919 and U. S. Patent 5,149,537), cation surface active agent, for example ethyleneoxide additive long chain amine condensate and qurternary ammonium compounds such as cetyltrimethylammonium bromide or dodecylmethylammonium bromide, anion surface active agent, for example salt of alkylbenzene sulfonate, salt of N-acyl-n-alkyl tauric acid and salt of α-olefinsulfonate, or non-ionic surface active agent, for example polyoxyalkylene higher alcohol ether and polyalkylene alkylphenols (Japanese Patent Unexamined Publication No. 4-247034) have been reported.

A preparation for transmucosal administration using absorption promotors acting through nasal mucosa or rectal mucosa, for example salt of glycyrrhizic acids such as diammonium glycyrrhizic acid and alkaline salt of glycyrrhizic acid (mono- or di-sodium, or mono- or di-potassium salt) (Japanese Patent Unexamined Publication No. 2-42027 and ibid. No. 3-5427), cyclodextrins such as α-cyclodextrin, β-cyclodextrin, γ-cyc1odextrin, mono- or di-methylated cyclodextrin ( α-, β- or γ-) (Japanese Patent Unexamined Publication No. 58-189118 and EP-A-No. 94157), O-acyl-L-carnitines having acyl of $C_{8\text{-}18}$ (Japanese Patent Unexamined Publication No. 63-10735 and EP-A-No. 215697), or chelating agents, polyacrylate gel and sodium capric acid (U. S. Patent 4,476,116) has been reported.

Azacycloalkane derivatives of the formula 〔1〕

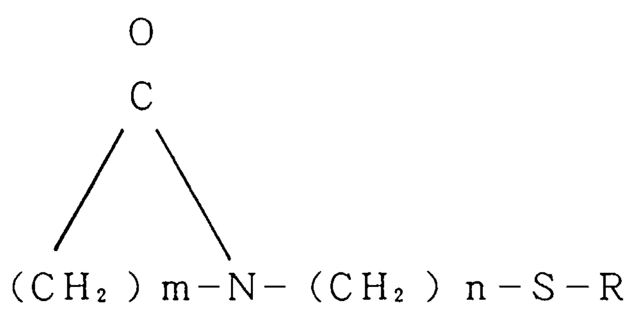

〔1〕

wherein R is an alkyl, m is an integer of 2 - 4 and n is an integer of 1 - 15, provided that R is an alkyl with a carbon number of 5 - 11 in case where n is 1 - 3 was known as the superior absorption promotor (Japanese Patent Unexamined Publication No. 62-238261).

Examples of absorption promotors such as cholic acids and fusidic acid derivatives are known [J. Japan. Diab. Soc., 20 (2); 146 - 152, 1977, Proc. Natl. Acad. Sci., U.S.A. 82 (21); 7419 - 7423, 1985 and Pharm Res., 9 (1); 52 - 57, 1992]. However these preparations are not satisfactory due to inferior absorbability and local irritation and are yet practically employed. Accordingly. superior absorbability of peptide is a key to the practical use.

PROBLEMS TO BE SOLVED BY THE INVENTION

The present invention relates to a preparation for transmucosal administration having superior absorbability of physiologically active peptide through nasal mucosa, oral mucosa, pulmonary mucosa, rectal mucosa, vaginal mucosa, ocular mucosa and digestive tract mucosa in order to exhibit sufficient pharmacological effects, without detrimental action on mucosa.

MEANS FOR SOLVING THE PROBLEMS

We have found that, as a result of extensive studies for development of preparation for transmucosal administration containing physiologically active peptide with superior absorbability and safety and less side-effect, a combination of the known absorption promotor and the vasodilator such as calcium channel blocker or prostaglandin E1, isosorbide dinitrateor nitroglycerin showed unexpectedly improved increased absorbability. The present invention has been completed by the above facts.

An object of the present invention is to provide a preparation for transmucosal administration containing physiologically active peptide comprising of a combination of physiologically active peptide and a known absorption promotor and a vasodilator. This is a completely unexpected result presumed from the prior arts because of being known nothing or few activity of vasodilator on absorption promoting action through mucosa.

The absorption promotor used in the present invention is a general term for changing biomembrane permeabiliiy, increasing tip absorbalility and increasing up bioavailability of the drugs. and has promoting action for absorption of physiologically active peptide on nasal mucosa or rectal mucosa. The absorption promotor used in the present invention exhibits improvement in absorbability at above 200 %, preferably above 500 %, in case of physiologically active peptide insulin absorption from nasal mucosa or rectal mucosa as compared with absorbability without such the absorption promotor.

Examples of the absorption promotor are known absorption promotor having absorption promoting action for physiologically active peptide on nasal mucosa or rectal mucosa. Such the examples are compound having surface active agent action, for example sodium taurocholic acid, sodium cholic acid, sodium deoxycholic acid, sodium chenodeoxycholic acid, lysine chenodeoxycholic acid, sodium glycocholic acid, sodium glycodeoxycholic acid and lysine taurocholic acid, cation surface active agent, for example ethyleneoxide additive long chain amine condensate and tertiary ammonium compounds such as cetyltrimethylammonium bromide or dodecylmethylammonium bromide, anion surface active agent, for example salt of alkylbenzene sulfonate, salt of N-acyl-n-alkyl tauric acid and salt of α-olefinsulfonate, non-ionic surface active agent, for example polyoxyalkylene higher alcohol ether and polyalkylene alkylphenols, salt of glycyrrhizic acids such as diammonium glycyrrhizic acid and alkaline salt of glycyrrhizic acid (mono- or di-sodium, or mono- or di-potassium salt), cyclodextrins such as α-cyclodextrin, β-cyclodextrin, γ-cyclodextrin, mono- or di-methylated cyclodextrin (α-, β- or γ-), O-acyl-L-carnitines having acyl of C $_{8\text{-}18}$ , or chelating agents, polyacrylate gel and sodium capric acid.

Further preferable examples of absorption promotors are, for example one or more compound selected from the group consisting of salt of cholic acid, salt of fusidic acid, salt of glycyrrhizic acid, salt of O-acyl-L-carnitin, phospholipids, non-ionic surface active agents, cyclodextrins, higher fatty acids, 1-alkyl-2-pyrrolidone derivatives, 1-dodecylazacyclo heptane-2-one (Azone), bacitracin. sodium azulene sulfonic acid and azacycloalkane derivatives of the formula 〔1〕

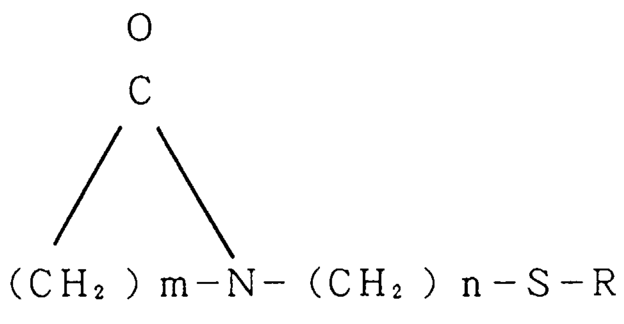

〔1〕

wherein R is an alkyl, m is an integer of 2 - 4 and n is an integer of 1 - 15, provided that R is an alkyl with a carbon number of 5 - 11 in case wherein n is 1 - 3.

Examples of cholic acid salts are one or more compounds selected from the group consisting of sodium taurocholic acid, sodium glycocholic acid and sodium deoxycholic acid.

Examples of fusidic acid salts are one or more compounds selected from the group consisting of sodium fusidic

acid and sodium tauro 24, 25-dihydrofusidic acid.

Examples of glycyrrhizic acid salts are one or more compounds selected from the group consisting of sodium glycyrrhizic acid and sodium 3-succinyloxyglycyrrhizic acid (carbenoxolon).

Examples of O-acyl-L-carnitin salts are, for example O-acyl-L-carnitins of acyl C $_{8-18}$, preferably O-octanoyl-L-carnitin hydrochloride, O-lauroyl-L-carnitin hydrochloride and O-palmitoyl-L-carnitin hydrochloride.

Examples of phospholipids are one or more compounds selected from the group consisting of phosphatidylcholine (lecithin), lysophosphatidyl choline (lysolecithin) and lysophosphatidylglycerol.

Examples of non-ionic surface active agents are one or more compounds selected from the group consisting of polyoxyalkylene higher alcohol ethers, polyoxyalkylene alkylphenols and sucrose fatty acid esters, and are preferably polyoxyethylene (9) lauryl ether (Laureth-9) and polyoxyethylene (24) cholesterylether (Choleth-24).

Examples of cyclodextrins are one or more compounds selected from the group consisting of $\alpha$-cyclodextrin, $\beta$-cyclodextrin, $\gamma$-cyclodextrin, and di-methyl- $\beta$-cyclodextrin.

Examples of higher fatty acids are one or more compounds selected from the group consisting of C $_{16-20}$ fatty acids and are preferably higher C $_{18}$ unsaturated fatty acid of oleic acid. linoleic acid and lino-lenic acid.

Examples of 1-alkyl-2-pyrrolidone derivatives are one or more compounds selected from the group consisting of compound having C $_{4-12}$ alkyl which are one or more compounds selected from the group consisting of butyl, pentyl, hexyl, heptyl, octyl, nonyl, decyl, undecyl and dodecyl.

Examples of R in azacycloalkane derivative 〔1〕 are straight or branched alkyl such as methyl, ethyl, propyl, butyl, pentyl, hexyl, heptyl, octyl, nonyl, decyl, undecyl, dodecyl, tridecyl, tetradecyl, pentadecyl, hexadecyl, heptadecyl, octadecyl, nonadecyl or eicodecyl, and preferably 1 - [2-(decylthio) ethyl] azacyclopentane-2-one (generic name: pyrothiodecane, oily substance), wherein R is C $_{10}$ alkyl, m is 2 and n is 2 in the formula 〔1〕.

Any compounds having absorption promoting activity on mucosa can be included in the present invention, and are not limited in the above.

Absorption promotor used in the present invention is preferably high safety and is 1- [2-(decylthio) ethyl] azacyclopentane-2-one, glycocholic acid, lysolecithin or sodium azulene sulfonic acid.

The most preferable absorption promotor used in preparation for transmucosal administration of the present invention is 1 - [2-(decylthio) ethyl] azacyclopentane-2-one or lysolecithin. Amount of these compounds in the preparation is 0.01 - 5 weight %.

Emulsion containing azacycloalkane derivative of the formula 〔1〕, preferably 1 - [2-(decylthio) ethyl] azacyclopentane-2-one, preferably contains 0.01 - 10 % (w/v), preferably 0.1 - 5 % (w/v) of glycyrrhizin or salt thereof. such as 2-potassium salt.

Vasodilators used in the present invention are compound of molecular weight 200 - 700 such as calcium channel blocker. Generally, calcium channel blocker shows vasodilating action and prolonged action of atrioventricular conduction time by suppressing influx of Ca into cells to show effect on hypertension and arrhythmia and is used for treatment of various circulatory diseases.

Examples thereof are benzodiazepine derivative such as diltiazem hydrochloride, phenylalkylamine derivative such as verapamil hydrochloride and bepridil hydrochloride, dihydropyridine derivative such as nifedipine hydrochloride, nicardipine hydrochloride and nimodipine hydrochloride, piperazine derivative such as cinnarizine and flunarizine and fasudil hydrochloride.

Other strong vasodilators are isosorbide dinitrate, nitroglycerin and prostaglandin E1. Isosorbide dinitrate and nitroglycerin have been used in the form of injection, peroral and tape preparation for treatment of ischemic heart disease and angina, Mechanisms of action thereof are direct action on vascular smooth muscles to show vasodilating action, and are to expand coronary artery to reduce coronary resistance as well as to expand collateral pathway to increase oxygen supply in ischemic cardiac muscle to improve cardiac function. Prostaglandin E1 has strong vasodilating activity and platelet aggregating inhibitory activity and is clinically used for treatment of hemostatic ulcer complicated with chronic arterial occulsive disease.

Vasodilator used in the present invention are not limited within the scope hereinabove, and any compounds having vasodilating action can be used in the present invention.

Compound having vasodilating activity used in the present invention is generally commercially available pharmaceuticals used for treatment of circulatory diseases, and is added in preparation for local administration in mucosa. In this case, amount of addition of vasodilator as an additive is not limited if such vasodilator shows effective absorption of physiologically active peptide, and is preferably below 1/2 amount of minimum effective dose of the vasodilator, more preferably below 1/5 amount. For example, a minimum usual dose of diltiazem hydrochloride is 10 mg and is preferably used as an additive of the present invention below 1/2 amount thereof, more preferably below 1/5 amount. A minimum usual dose of prostaglandin E1 injection is 20 $\mu$g, and is preferably used as an additive of the present invention below 1/2 amount (10 $\mu$g) thereof and more preferably below 1/5 amount (4 $\mu$g). Minimum limited amount may be above 0.1 $\mu$g, preferably above 1 $\mu$g in a dosage regimen.

Physiologically active peptide used in the present invention are peptide consisting of more than 3 amino acids, of

which molecular weight about 300 - 10,000. Examples of the peptide are insulin, calcitonin, human PTH (1 - 34), calcitonin gene related peptide (CGRP), angiotensin II, vasopressin, desmopressin acetate, buserelin acetate, goserelin acetate, nafarelin acetate, leuprorelin acetate, somatostatin, glucagon, oxytocin, secretin, LH - RH, ACTH, TRH, TSH, ANP or derivatives containing synthetic or semisynthetic compound thereof.

Calcitonin includes, for example natural calcitonin such as eel calcitonin, salmon calcitonin, human calcitonin, porcine calcitonin and chicken calcitonin, and semisynthetic calcitonin such as ASU $^{1-7}$ eel calcitonin (elcatonin) or ASU $^{1-7}$ chicken calcitonin. Examples of insulin are human insulin, porcine insulin or bovine insulin.

Most preferable peptides used in the present invention are elcatonin and human insulin.

Formulation of the preparation for transmucosal administration of the present invention is explained hereinbelow.

Amount of physiologically active peptide in the preparation of the present invention can be selected by an activity of the peptide and necessity of dosage amount, and depends on absorbability of the peptide from nasal mucosa. For example, preferable concentration of aqueous preparation of physiologically active peptide in the preparation for transmucosal administrationis 0.000001 - 5 % (w/v) and more preferably 0.00001 - 1% (w/v).

The preparation for transmucosal administration of the present invention can be applied on nasal mucosa, oral mucosa, pulmonary mucosa, rectal mucosa, vaginal mucosa and ocular mucosa. In the preparation. formula can be a solution, solid or semi-solid form, and is preferably aqueous preparation for spray or drop, or suppositories. Aqueous or suppository preparation contains water soluble or oily base ingredients such as distilled water, glycerin, propylene glycol, Witepsol, cacao butter, soybean oil, neutral fatty acid triglyceride or polyvinylpyrrolidone.

Aqueous preparation can be prepared by containing physiologically active peptide, one or more absorption promotors selected from the above and one or more vasodilators selected from the above, and if required adding suitable amount of pH adjusting agent, tonicity agents, preservatives, stabilizers, solubilizers or emulsifiers, and solubilizing or emulsifying thereof with addition of suitable amount of distilled water to prepare solution or emulsion. If stability is required, solid preparation can be prepared by lyophilized or spray dried.

A pH of the preparation can be selected not to affect stability of physiologically active peptide, within less damage to nasal mucosa and without precipitation. Preferable pH is generally pH 4 - 8. Examples of pH adjusters are sodium hydroxide, potassium hydroxide, sodium carbonate, sodium hydrogen carbonate, hydrochloric acid, sulfric acid or buffers such as phosphate, acetate, lactate or citrate. Osmotic pressure is preferably isotonic.

Examples of tonicity agent are glycerin, sodium chloride, manitol and glucose. Pharmacologically acceptable preservatives can be used. Example thereof are methyl parahydroxybenzoate, ethyl parahydroxybenzoate, propyl parahydroxybenzoate, butyl parahydroxybenzoate, benzalkonium chloride, benzethonium chloride, phenylethyl alcohol, chlorobutanol, phenol, cresol, thimerosal, dehydroacetic acid and sorbicacid, Preferable concentration of the preservatives depends on the selected preservative and is 0.01 % - 2 % (w/v).

Production of preparation of the present invention can be performed by conventional means. Aqueous preparation can be prepared, for example by adding water for injection to insulin. lysolecithin, prostaglandin E1 and additives hereinbefore with stirring to solubilize, and adjusting pH by adding pH adjuster such as sodium hydroxide or hydrochloric acid. The solution is filtered aseptically through, for example 0.22 μm membrane filter, and is filled in vials (Sanvagon Corp, U-SAVE) to prepare aqueous preparation.

Dosage depends on a purpose of administration. Preparation for nasal administration can be administered in human using constant sprayer (0.05 - 0.1 ml per one pushing) into one side or both nasal cavity, 1 - 3 times in a day.

Preparation for administration through rectal mucosa or vaginal mucosa can be prepared by adding necessry amount of Witepsol, cacao butter, macrogol, propylene glycol or glycerin.

Administration route of the preparation of the present invention is in general performed by spray drying into mucosa for systemic action. The preparation of the present invention adheres to the wide area of mucosa and is completely permeate through mucosa to distribute peptide systemically. Accordingly, the preparation containing peptide for transmucosal administration of the present invention has no problems of pains in patients caused by injection, and autoadministration can be possible.

Also the preparation can be prepared for transmucosal administration through nasal mucosa, pulmonary mucosa, rectal mucosa, vaginal mucosa and ocular mucosa.

## BRIEF EXPLANATION OF THE DRAWINGS

Fig. 1 : Profile of serum levels after nasal administration of elcatonin.
Fig. 2 : Absorption ratio (%) on subcutaneous injection of elcatonin (AUC).
Fig. 3 : Profile of serum levels after nasal administration of human insulin.
Fig. 4 : Profile of serum levels after nasal administration of human insulin.
Fig. 5 : Profile of serum levels after nasal administration of LH - RH.

## EMBODIMENTS OF THE INVENTION

Following examples and referential examples illustrate the present invention but are not construed as limiting. Further following experimental examples illustrate effect of the present invention.

## REFERENTIAL EXAMPLE 1

Sodium glycocholic acid (SGC : Sigma Corp., U.S.A.) 5 mg was dissolved in isotonic phosphate buffer solution pH 6.0, 1 ml. The solution 1 ml was added in elcatonin 400 units/vial (lyophilized) to prepare elecatonin 400 units/ml solution (control 1).

## REFERENTIAL EXAMPLE 2

Diltiazem hydrochloride (DTZ : Sigma Corp., U.S.A.) 10 mg was dissolved in isotonic phosphate buffer solution pH 6.0, 2 ml. The solution 1 ml was added in elcatonin 400 units/vial (lyophilized) to prepare elecatonin 400 units/ml solution (control 2).

## EXAMPLE 1

Diltiazem hydrochloride (DTZ : Sigma Corp., U.S.A.) 10 mg was dissolved in 0.5 % sodiuim glycocholic acid aqueous solution (pH 6.0 isotonic phosphate buffer solution) 2 ml to prepare solution containing 0.5 % SGC and 0.5 % DTZ. The solution 1 ml was added in elcatonin 400 units/vial (lyophilized) to prepare elecatonin 400 units/ml solution.

## EXAMPLE 2

Verapamil hydrochloride (VP : Sigma Corp., U.S.A.) 10 mg was dissolved in 0.5 % sodiuim glycocholic acid solution 2 ml, which was prepared by dissolving previously N-vinyl-2-pyrrolidon (Wako Pure Chem. Co., Japan) 50 mg and sodium glycocholic acid (SGC : Sigma Corp., U.S.A.) 5 mg in isotonic phosphate buffer pH 6.0, 1 ml, to prepare solution containing 0.5 % SGC and 0.5 % VP. The solution 1 ml was added in elcatonin 400 units/vial (lyophilized) to prepare elecatonin 400 units/ml solution.

## EXAMPLE 3

Bepridil hydrochloride (BP : Sigma Corp., U.S.A.) 10 mg was dissolved in 0.5 % sodiuim glycocholic acid solution 2 ml, which was prepared by dissolving previously N-vinyl-2-pyrrolidon (Wako Pure Chem. Co., Japan) 100 mg and sodium glycocholic acid (SGC : Sigma Corp., U.S.A.) 5 mg in isotonic phosphate buffer solution pH 6.0, 1 ml, to prepare solution containing 0.5 % SGC and 0.5 % BP. The solution 1 ml was added in elcatonin 400 units/vial (lyophilized) to prepare elecatonin 400 units/ ml solution.

## EXAMPLE 4

Fusidil hydrochloride (FS : Asahi Chem. Co., Japan) 10 mg was dissolved in 0.5 % sodiuim glycocholic acid aqueous solution (pH 6.0 isotonic buffer solution) 2 ml to prepare solution containing 0.5 % SGC and 0.5 % FS. The solution 1 ml was added in elcatonin 400 units/vial (lyophilized) to prepare elecatonin 400 units/ml solution.

## EXPERIMENT 1

In vivo absorption of elcatonin by rats :

[in vivo experiment]

Wistar rats, male (Japan SLC, body weight 200 - 250 g), fasting overnight, 4 rats in a group, were anesthetized by pentobarbital (Nembutal inj. Dainippon Pharm. Co., Japan).

Polyethylene tube was inserted into the trachea by incised in the corvex. Tube was inserted into postnasal space after esophageal incision. Administration was conducted by pouring drug solution 25 μl prepared in use using micropipette into the right naris. Blood sample 200 μl was collected from femoral vein time dependently before and after administration of drug. Blood sample was centrifuged (15,000 rpm/10 min./5°C) and stored at -30 °C before analysis.

Elcatonin 20 units/ml (in physiological saline) 0.25 ml was subcutaneously administered for comparison with

absorption rate (comparison 1).

QUANTITATIVE METHOD

Blood level was assayed by RIA.

RESULT

Time dependent curve of serum elcatonin is shown in Fig. 1.

Significant increase in serum elcatonin level was observed in the administration group (▲-▲) in example 1, in which elcatonin, 0.5 % sodium glycocholic acid and 0.5 % diltiazem hydrochloride were added, as compared with control group 1 containing elcatonin and 0.5 % sodium glycocholic acid (■-■). About 3.6 folds increase in elcatonin absorption through nasal mucosa were observed by comparing area under the curve (AUC) of the experimental group with the control 1. Almost no absorption through nasal mucosa was observed in the administered group of control 2 (●-●) containing elcatonin and diltiazem hydrochloride. Therefore, diltiazem hydrochloride per se has no absorption promoting action. In conclusion, combination of absorption promotor sodium glycocholic acid and diltiazem hydrochloride, a vasodilator of calcium blocker, shows excellent absorption promoting action, Further, administration group of varapamil hydlrochloride (Fig. 1, ○ -○ ), bepridil hydrochloride (Fig. 1, △-△) and fasudil hydlrochloride (Fig. 1, □-□) in example 2, 3 and 4 showed increased absorption as same as of in diltiazem hydlrochloride.

Fig. 2 shows increased absorption promoting action of various calcium channel blockers.

Absorption rate is calculated by using area under the curve (AUC) in the comparison 1 from the following equation.

$$\text{Absorption rate (\%)} = \frac{\text{AUC (I.N.)}}{\text{AUC (S.C.)}} \times \frac{\text{dose (S.C.)}}{\text{dose (I.N.)}} \times 100$$

wherein I. N. : nasal administraion and S.C. : subcutaneous administration. As clearly shown in Fig. 2, absorption of elcatonin was enhanced in above 2 fold through nasal mucosa by administering with absorption promotor sodium glycocholate and calciuim channel blocker as compared with sodium glycocholic acid per se.

REFERENTIAL EXAMPLE 3

A r-human insulin (gene manipulation product from yeast : specific activity 26 units/mg : Boehringer Mannheim Co., Germany) 10 units/vial (lyophilized) was dissolved in isotonic phosphate buffer solution pH 7.4, 1 ml to prepare human insulin solution 10 units/ml (control 3).

REFERENTIAL EXAMPLE 4

L- α-lysolecithin (LPC : Sigma Corp., U.S.A.) 5 mg was dissolved in isotonic phosphate buffer solution pH 7.4, 1 ml to prepare solution containing 0.5 % LPC. The solution 1 ml was added in r-human insulin 10 units/vial (lyophilized) to prepare r-human insulin 10 units/ml solution (control 4).

EXAMPLE 5

Diltiazem hydrochloride (DTZ : Sigma Corp., U.S.A.) 10 mg was dissolved in 0.5 % L-α-lysolecithin solution (isotonic phosphate buffer solution pH 6.0) 2 ml to prepare solution containing 0.5 % LPC and 0.5 % DTZ. The solution 1 ml was added in r-human insulin 10 units/vial (lyophilized) to prepare human insulin 10 units/ml solution.

EXAMPLE 6

Prostaglandin R20 for injection (Prostaglandin E1 : PGE1 : Ono Pharm. Co., Japan) 20 μg was dissolved in 0.5 % L- α-lysolecithin (LPC) solution (isotonic phosphate buffer solution pH 7.4) 2 ml to prepare solution containing 0.5 % LPC and 0.001 % PGE1. The solution 1 ml was added in r-human insulin 10 units/vial (lyophilized) to prepare human insulin 10 units/ml solution.

EXPERIMENT 2

In vivo absorption of insulin by rats :

[in vivo experiment]

Wistar rats, male (Japan SLC, body weight 200 - 250 g), fasting overnight, 4 rats in a group, were anesthetized by pentobarbital (Nembutal inj. Dainippon Pharm. Co., Japan).

Polyethylene tube was inserted into the trachea by incised in the corvex to maintain the trachea. Tube, edge of which was closed by cotton, was inserted into postnasal space after esophageal incision in order to prevent leak of drug solution from the nasal cavity to the esophagus. Administration was conducted by pouring drug solution 25 μl prepared in use usingmicropipette into the right naris. Blood sample 200 μl was collected from the femoral vein time dependently before and after administration of drug. Blood sample was centrifuged (15, 000 rpm/10 min./5°C) and stored at -30 °C before analysis.

QUANTITATIVE METHOD

Blood level was assayed by using human insulin assay reagent (Boehringer Mannheim Co., Germany), an EIA of one step sandwitch method using 2 types of monoclonal antibody.

RESULT

Time dependent curve of serum insulin concentration is shown in Fig. 3. In Fig. 3, insulin solution administered group, control 3 (●-●), shows almost no absorption through the nasal mucosa. Significant increase in absorption was observed in a group added with insulin. 0.5 % diltiazem hydrochloride and 0.001 % PGE1 as compared with a group of control 4 (○-○) added with insulin and 0.5 % L-α-lyso-lecithin (LPC). Area under the curve (AUC) of administered group of the preparation in example 5 (■-■) added with 0.5 % diltiazem hydrochloride and that of the preparation in example 6 (▲-▲) added with 0.001 % PGE1, increased about 1.7 fold and 1.8 fold, respectively, in absorption of insulin through the nasal mucosa.

EXAMPLE 7

In 1 ml of preparation for transmucosal administration :

| | |
|---|---|
| 1. elcatonin | 1000 units |
| 2. sodium glycocholic acid | 5 mg |
| 3. diltiazem hydrochloride | 5 mg |
| 4. glycerin | 22 mg |
| 5. methyl parahydroxy benzoic acid | 1 mg |
| 6. hydrochloric acid/sodium hydroxoide ad libitum for adjusting to pH 5.5 | |
| 7. water for injection | total 1 ml |

The preparation containing the above was prepared. The solution was filtered aseptically (0.22 μm membrane filter) and packed aseptically 3 ml thereof in a vial proper to quantitative mechanical spray for nasal administration to prepare the product. This product contains elcatonin 1000 units/ml and constant spray administration of 100 units of elcatonin can be performed by one pushing on adaptor.

EXAMPLE 8

In 1 ml of preparation for transmucosal administration :

| | |
|---|---|
| 1. elcatonin | 1000 units |
| 2. L- α-lysolecithin | 5 mg |

(continued)

| | |
|---|---|
| 3. prostaglandin E1 | 10 μg |
| 4. glycerin | 22 mg |
| 5. methyl parahydroxy benzoic acid | 1 mg |
| 6. hydrochloric acid/sodium hydroxoide ad libitum for adjusting to pH 5.5 | |
| 7. water for injection | total 1 ml |

The preparation containing the above was prepared. The solution was filtered aseptically (0.22 μm membrane filter) and packed aseptically 3 ml thereof in a vial proper to quantitative mechanical spray for nasal administration to prepare the product. This product contains elcatonin 1000 units/ml and constant spray administration of 100 units of elcatonin can be performed by one pushing on adaptor.

EXAMPLE 9

In 1 ml of preparation for transmucosal administration :

| | |
|---|---|
| 1. elcatonin | 1000 units |
| 2. L- α-lysolecithin | 5 mg |
| 3. isosorbide dinitrate | 0.1 mg |
| 4. glycerin | 17.6 mg |
| 5. D-sorbitol | 10 mg |
| 6. hydrochloric acid/sodium hydroxoide ad libitum for adjusting to pH 5.5 | |
| 7. water for injection | total 1 ml |

The preparation containing the above was prepared. The solution was filtered aseptically (0.22 μm membrane filter) and packed aseptically 3 ml thereof in a vial proper to quantitative mechanical spray for nasal administration to prepare the product. This product contains elcatonin 1000 units/ml and constant spray administration of 100 units of elcatonin can be performed by one pushing on adaptor.

EXAMPLE 10

In 1 ml of preparation for transmucosal administration :

| | |
|---|---|
| 1. r-human insulin | 100 units |
| 2. sodium glycocholic acid | 5 mg |
| 3. diltiazem hydrochloride | 5 mg |
| 4. glycerin | 22 mg |
| 5. methyl parahydroxy benzoic acid | 1 mg |
| 6. hydrochloric acid/sodium hydroxoide ad libitum for adjusting to pH 6.0 | |
| 7. water for injection | total 1 ml |

The preparation containing the above was prepared. The solution was filtered aseptically (0.22 μm membrane filter) and packed aseptically 3 ml thereof in a vial proper to quantitative mechanical spray for nasal administration to prepare

the product. This product contains r-human insulin 100 units/ml and constant spray administration of 10 units of human insulin can be performed by one pushing on adaptor.

EXAMPLE 11

In 1 ml of preparation for transmucosal administration :

| | |
|---|---|
| 1. r-human insulin | 100 units |
| 2. L- α-lysolecithin | 5 mg |
| 3. prostaglandin E1 | 10 μg |
| 4. glycerin | 22 mg |
| 5. benzalkonium chloride | 0.1 mg |
| 6. hydrochloric acid/sodium hydroxoide ad libitum for adjusting to pH 6.0 | |
| 7. water for injection | total 1 ml |

The preparation containing the above was prepared. The solution was filtered aseptically (0.22 μm membrane filter) and packed aseptically 3 ml thereof in a vial proper to quantitative mechanical spray for nasal administration to prepare the product. This product contains r-human insulin 100 units/ml and constant spray administration of 10 units of human insulin can be performed by one pushing on adaptor.

EXAMPLE 12

In 1 ml of preparation for transmucosal administration :

| | |
|---|---|
| 1. r-human insulin | 100 units |
| 2. L- α-lysolecithin | 5 mg |
| 3. isosorbide dinitrate | 0.1 mg |
| 4. glycerin | 17.6 mg |
| 5. benzalkonium chloride | 0.1 mg |
| 6. D-sorbitol | 10 mg |
| 7. hydrochloric acid/sodium hydroxoide ad libitum for adjusting to pH 6.0 | |
| 8. water for injection | total 1 ml |

The preparation containing the above was prepared. The solution was filtered aseptically (0.22 μm membrane filter) and packed aseptically 3 ml thereof in a vial proper to quantitative mechanical spray for nasal administration to prepare the product. This product contains r-human insulin 100 units/ml and constant spray administration of 10 units of human insulin can be performed by one pushing on adaptor.

REFERENTIAL EXAMPLE 5

A solution containing pyrothiodecane (1-[2-(decylthio)ethyl]-azacyclopentane-2-one (Hisamitsu Pharm. Co., Japan) 5 mg as an absorption promotor, dipotassium glyclyrrhizic acid 10 mg as an emulsifier and glycerin 22 mg as an isotonic agent in distilled water was prepared and homogenized by means of ultrasonication, a solution of which was adjusted by adding 1 N sodium hydroxide to pH 6.0. A volume of the solution was adjusted to measure up to 1 ml to prepare 0.5 % pyrothiodecane solution. The solution thereof 1 ml was added in r-human insulin 10 units/vial (lyophilized) to prepare liquid preparation of human insulin 10 units/ml (control 5).

REFERENTIAL EXAMPLE 6

A solution containing prostaglandin E1 (PGE1 : prostaglandin R20 for injection, Ono Pharm. Co., Japan) 0.05 mg and glycerin 25.7 mg as anisotonic agent in distilled water, was prepared and adjusted to pH 6.0 by adding 1N-sodium hydroxide. The solution was measured up to 1 ml to prepare 0.005 % PGE1 solution. The solution 1 ml was added in r-human insulin 100 units/vial (lyophilized) to prepare liquid preparation of insulin 10 units/ml (control 6).

EXAMPLE 13

A solution containing pyrothiodecane 5 mg as an absorption promotor, dipotassium glyclyrrhizic acid 10 mg as an emulsifier, glycerin 22 mg as an isotonic agent and vasodilator prostaglandin E1 (PGE1 : prostaglandin R20 for injection) 0.01 mg in distilled water was prepared and homogenized by means of ulltrasonication, a solution of which was adjusted by adding 1 N sodium hydroxide to pH 6.0. A volume of the solution was adjusted to measure up to 1 ml to prepare 0.5 % pyrothiodecane and 0.001 % PGE1 solution. The solution thereof 1 ml was added in r-human insulin 10 units/vial (lyophilized) to prepare liquid preparation of human insulin 10 units/ml.

EXAMPLE 14

A solution containing pyrothiodecane 5 mg as an absorption promotor, dipotassium glyclyrrhizic acid 10 mg as an emulsifier. glycerin 17.6 mg as an isotonic agent and vasodilator isosorbide dinitrate (ISDN : Nitorol inj., Eizai Co., Japan) 0.1 mg in distilled water was prepared and homogenized by means of ulltrasonication, a solution of which was adjusted by adding 1 N sodium hydroxide to pH 6.0. A volume of the solution was adjusted to measure up to 1 ml to prepare 0.5 % pyrothiodecane and 0.01 % ISDN solution. The solution thereof 1 ml was added in r-human insulin 10 units/vial (lyophilized) to prepare liquid preparation of human insulin 10 units/ml.

EXAMPLE 15

A solution containing pyrothiodecane 5 mg as an absorption promotor, dipotassium glyclyrrhizic acid 10 mg as an emulsifier, glycerin 13.2 mg as an isotonic agent and vasodilator isosorbide dinitrate (ISDN : Nitorol inj., Eizai Co., Japan) 0.2 mg in distilled water was prepared and homogenized by means of ulltrasonication. a solution of which was adjusted by adding 1 N sodium hydroxide to pH 6.0. A volume of the solution was adjusted to measure up to 1 ml to prepare 0.5 % pyrothiodecane and 0.02 % ISDN solution. The solution thereof 1 ml was added in r-human insulin 10 units/vial (lyophilized) to prepare liquid preparation of human insulin 10 units/ml.

EXAMPLE 16

A solution containing pyrothiodecane 5 mg as an absorption promotor, dipotassium glyclyrrhizic acid 10 mg as an emulsifier. glycerin 17.6 mg as an isotonic agent and vasodilator nitroglycerin (Millisrol inj., Nippon Kayaku Co., Japan) 0.1 mg in distilled water was prepared and homogenized by means of ulltrasonication, a solution of which was adjusted by adding 1 N sodium hydroxide to pH 6.0. A volume of the solution was adjusted to measure up to 1 ml to prepare 0.5 % pyrothiodecane and 0.01 % nitroglycerin solution. The solution thereof 1 ml was added in r-human insulin 10 units/vial (lyophilized) to prepare liquid preparation of human insulin 10 units/ml.

EXPERIMENT 3

In vivo absorption of insulin by rats (2) :

[in vivo experiment]

Wistar rats, male (Japan SLC, body weight 200 - 250 g), fasting overnight, 4 rats in a group, were anesthetized by pentobarbital (Nembutal inj. Dainippon Pharm. Co., Japan).

Polyethylene tube was inserted into the trachea by incised in the corvex to maintain the trachea. Tube, edge of which was closed by cotton, was inserted into postnasal space after esophageal incision in order to prevent leak of drug solution from the nasal cavity to the esophagus. Administration was conducted by pouring drug solution 25 μl prepared in use using micropipette into the right naris. Blood sample 200 μl was collected from the femoral vein time dependently before and after administration of drug. Blood sample was centrifuged (15,000 rpm/10 min./5°C) and stored at -30 °C before analysis.

QUANTITATIVE METHOD

Blood level was assayed by using human insulin assay reagent (Boehringer Mannheim Co., Germany), an EIA of one step sandwitch method using 2 types of monoclonal antibody.

RESULT

Time dependent curve of serum insulin concentration is shown in Fig. 4. In Fig. 4, insulin solution administered group. control 3 (●-●), shows almost no absorption through the nasal mucosa. Similarly, a solution containing insulin and 0.005 % PGE1 (control 6) administered group (○-○) shows almost no absorption. Significant increase in absorption of insulin from nasal mucosa was observed in the group of the preparation of example 13 (■-■) added with insulin, 0.5 % pyrothiodecane and 0.001 % PGE1, the group of the preparation of example 14 ( ▲-▲) added with 0.01 % ISDN, the group of the preparation of example 15 (□-□) added with 0.02 % ISDN, and the group of the preparation of example 16 added with 0.01 % nitroglycerin (+ - +) as compared with the conventional preparation of the group of control 5 (△-△) containing insulin and 0.5 % pyrothiodecane. Area under the curve (AUC ; 0 - 2 hr.) of these administered groups of the preparation increased significantly about 1.7 fold, 1.9 fold, 2.0 folds and 2.4 folds, respectively, as compared with the administered group of the control 6.

EXAMPLE 17

In 1 ml of preparation for transmucosal administration :

| | |
|---|---|
| 1. r-human insulin | 100 units |
| 2. pyrothiodecane | 5 mg |
| 3. PGE1 | 0.005 mg |
| 4. dipotassium glycyrrhizic acid | 10 mg |
| 5. glycerin | 22 mg |
| 6. benzalkonium chloride | 0.1 mg |
| 7. hydrochloric acid/sodium hydroxoide ad libitum for adjusting to pH 6.0 | |
| 8. water for injection | total 1 ml |

The preparation containing the above was prepared. The solution was filtered aseptically (0.22 μm membrane filter) and packed aseptically 3 ml thereof in a vial proper to quantitative mechanical spray for nasal administration to prepare the product. This product contains r-human insulin 100 units/ml and constant spray administration of 10 units of human insulin can be performed by one pushing on adaptor.

EXAMPLE 18

In 1 ml of preparation for transmucosal administration :

| | |
|---|---|
| 1. r-human insulin | 100 units |
| 2. pyrothiodecane | 5 mg |
| 3. PGE1 | 0.01 mg |
| 4. dipotassium glycyrrhizic acid | 10 mg |
| 5. glycerin | 22 mg |
| 6. benzalkonium chloride | 0.1 mg |
| 7. hydrochloric acid/sodium hydroxoide ad libitum for adjusting to pH 6.0 | |

(continued)

| | |
|---|---|
| 8. water for injection | total 1 ml |

The preparation containing the above was prepared. The solution was filtered aseptically (0.22 μm membrane filter) and packed aseptically 3 ml thereof in a vial proper to quantitative mechanical spray for nasal administration to prepare the product. This product contains r-human insulin 100 units/ml and constant spray administration of 10 units of human insulin can be performed by one pushing on adaptor.

EXAMPLE 19

In 1 ml of preparation for transmucosal administration :

| | |
|---|---|
| 1. r-human insulin | 100 units |
| 2. pyrothiodecane | 5 mg |
| 3. isosorbide dinitrate | 0.1 mg |
| 4. dipotassium glycyrrhizic acid | 10 mg |
| 5. glycerin | 17.6 mg |
| 6. benzalkonium chloride | 0.1 mg |
| 7. D-sorbitol | 10 mg |
| 8. hydrochloric acid/sodium hydroxoide ad libitum for adjusting to pH 6.0 | |
| 9. water for injection | total 1 ml |

The preparation containing the above was prepared. The solution was filtered aseptically (0.22 μm membrane filter) and packed aseptically 3 ml thereof in a vial proper to quantitative mechanical spray for nasal administration to prepare the product. This product contains r-human insulin 100 units/ml and constant spray administration of 10 units of human insulin can be performed by one pushing on adaptor.

EXAMPLE 20

In 1 ml of preparation for transmucosal administration :

| | |
|---|---|
| 1. r-human insulin | 100 units |
| 2. pyrothiodecane | 5 mg |
| 3. isosorbide dinitrate | 0.2 mg |
| 4. dipotassium glycyrrhizic acid | 10 mg |
| 5. glycerin | 13.2 mg |
| 6. benzalkonium chloride | 0.1 mg |
| 7. D-sorbitol | 20 mg |
| 8. hydrochloric acid/sodium hydroxoide ad libitum for adjusting to pH 6.0 | |
| 9. water for injection | total 1 ml |

The preparation containing the above was prepared. The solution was filtered aseptically (0.22 μm membrane filter) and packed aseptically 3 ml thereof in a vial proper to quantitative mechanical spray for nasal administration to prepare

the product. This product contains r-human insulin 100 units/ml and constant spray administration of 10 units of human insulin can be performed by one pushing on adaptor.

EXAMPLE 21

In 1 ml of preparation for transmucosal administration

| | |
|---|---|
| 1. r-human insulin | 100 units |
| 2. pyrothiodecane | 5 mg |
| 3. nitroglycerin | 0.1 mg |
| 4. dipotassium glycyrrhizic acid | 10 mg |
| 5. glycerin | 17.6 mg |
| 6. benzalkonium chloride | 0.1 mg |
| 7. D-mannitol | 10 mg |
| 8. hydrochloric acid/sodium hydroxoide ad libitum for adjusting to pH 6.0 | |
| 9. water for injection | total 1 ml |

The preparation containing the above was prepared. The solution was filtered aseptically (0.22 μm membrane filter) and packed aseptically 3 ml thereof in a vial proper to quantitative mechanical spray for nasal administration to prepare the product. This product contains r-human insulin 100 units/ml and constant spray administration of 10 units of human insulin can be performed by one pushing on adaptor.

REFERENTIAL EXAMPLE 7

A solution containing isosorbide (ISDN : Nitorol inj. Eizai Co., Japan) 0.1 mg and glycerin 25.7 mg an an isotonic agent in distilled water, was prepared and adjusted to pH 6.0 by adding 1 N-sodium hydroxide. The solution was measured up to 1 ml to prepare 0.01 % ISDN solution. The solution 1 ml was added in luteinizing hormone-releasing hormone (LH - RH, Sigma Corp., U.S.A.) 100 μg/vial (lyophilized) to prepare liquid preparation of LH - RH in 100 μg/ml (control 7).

REFERENTIAL EXAMPLE 8

A solution containing pyrothiodecane 5 mg as an absorption promotor, dipotassium glyclyrrhizic acid 10 mg as an emulsifier aid glycerin 22 mg as an isotonic agent in distilled water was prepared and homogenized by means of ulltrasonication, a solution of which was adjusted by adding 1 N sodium hydroxide to pH 6.0. A volume of the solution was adjusted to measure up to 1 ml to prepare 0.5 % pyrothiodecane solution. The solution thereof 1 ml was added in luteinizing hormone-releasing hormone (LH - RH, Sigma Corp., U.S.A.) 100 μg/vial (lyophilized) to prepare liquid preparation of LH - RH in 100 μg/ml (control 8).

EXAMPLE 22

A solution containing pyrothiodecane 5 mg as an absorption promotor, dipotassium glyclyrrhizic acid 10 mg as an emulsifier, glycerin 17.6 mg as an isotonic agent and vasodilator isosorbide dinitrate (ISDN : Nitorol inj., Eizai Co., Japan) 0.1 mg in distilled water was prepared and homogenized by means of ulltrasonication. a solution of which was adjusted by adding 1 N sodium hydroxide to pH 6.0. A volume of the solution was adjusted to measure up to 1 ml to prepare 0.5 % pyrothiodecane and 0.01 % ISDN solution. The solution thereof 1 ml was added in luteinizing hormone-releasing hormone (LH - RH, Sigma Corp., U.S.A.) 100 μg/vial (lyophilized) to prepare liquid preparation of LH - RH in 100 μg/ml.

EXPERIMENT 4

In vivo absorption of LH - RH by rats :

[in vivo experiment]

Wistar rats, male (Japan SLC, body weight 200 - 250 g), fasting overnight, 4 rats in a group, were anesthetized by pentobarbital (Nembutal inj. Dainippon Pharm. Co., Japan).

Polyethylene tube was inserted into the trachea by incised in the corvex to maintain the trachea. Tube, edge of which was closed by cotton, was inserted into postnasal space after esophageal incision in order to prevent leak of drug solution from the nasal cavity to the esophagus. Administration was conducted by pouring drug solution 10 μg/0.1 mg/kg prepared in use using micropipette into the right naris. Blood sample 200 μl was collected from the femoral vein time dependently beforeand after administration of drug. Blood sample was centrifuged (15,000 rpm/10 min./5 °C) and stored at -30 °C before analysis.

QUANTITATIVE METHOD

Blood level was assayed by using LH - RH assay reagent (Peninsula Corp., U.S.A.), an competitive EIA method.

RESULT

Time dependent curve of serum LH - RH concentration is shown in Fig. 5. In Fig. 5, the administered group of control 7 containing LH - RH and 0.01 % ISDN ( ●-●) shows almost no absorption through the nasal mucosa. Significant increase in absorption was observed in a group added with LH - RH, 0.5 % pyrothiodecane and 0.01 % ISDN of the present invention (■-■) as compared with a group of control 8 (▲-▲) containing conventional preparation of LH - RH and 0.5 % pyrothiodecane. Area under the curve (AUC ; 0-2 hr.) of the administered group of the present invention increased about 1.5 fold as compared with the AUC of the control 8.

EXAMPLE 23

In 1 ml of preparation for transmucosal administration :

| | |
|---|---|
| 1. LH - RH | 5 mg |
| 2. pyrothiodecane | 5 mg |
| 3. isosorbide dinitrate | 0.1 mg |
| 4. dipotassium glycyrrhizic acid | 10 mg |
| 5. glycerin | 17.6 mg |
| 6. benzalkonium chloride | 0.1 mg |
| 7. D-sorbitol | 10 mg |
| 8. hydrochloric acid/sodium hydroxoide ad libitum for adjusting to pH 6.0 | |
| 9. water for injection | total 1 ml |

The preparation containing the above was prepared. The solution was filtered aseptically (0.22 μm membrane filter) and packed aseptically 3 ml thereof in a vial proper to quantitative mechanical spray for nasal administration to prepare the product. This product contains LH - RH 5 mg/ml and constant spray administration of 0.5 mg of LH - RH can be performed by one pushing on adaptor.

REFERENTIAL EXAMPLE 9

Isosorbide dinitrate (ISDN : Nitorol inj., Eizai Co., Japan) 0.2 mg was dissolved in isotonic phosphate buffer solution pH 7.4, 1 ml to prepare solution to prepare a solution containing 0.02 % ISDN. The solution 1 ml was added in r-human

insulin 10 units/vial (lyophilized) to prepare human insulin 10 units/ml solution (control 9).

EXAMPLE 24

Isosorbide dinitrate (ISDN : Nitorol inj., Eizai Co., Japan) 0.1 mg dissolved in 0.5 % L-α-lysolecithin solution (isotonic phosphate buffer solution pH 7.4) 1 ml to prepare solution containing 0.5 % LPC and 0.01 % ISDN. The solution 1 ml was added in r-human insulin 10 units/vial (lyophilized) to prepare human insulin 10 units/ml solution.

EXAMPLE 25

Nitoroglycerin (Millisrol inj., Nihon Kayaku Co., Japan) 0.1 mg dissolved in 0.5 % L-α-lysolecithin solution (isotonic phosphate buffer solution pH 7.4) 1 ml to prepare solution containing 0.5 % LPC and 0.01 % nitroglycerin. The solution 1 ml was added in r-human insulin 10 units/vial (lyophilized) to prepare human insulin 10 units/ml solution.

EXPERIMENT 5

In vivo absorption of insulin by rats (3) :

[in vivo experiment]

Wistar rats, male (Japan SLC, body weight 200 - 250 g), fasting overnight, 4 rats in a group, were anesthetized by pentobarbital (Nembutal inj. Dainippon Pharm. Co., Japan).

Polyethylene tube was inserted into the trachea by incised in the corvex to maintain the trachea. Tube, edge of which was closed by cotton, was inserted into postnasal space after esophageal incision in order to prevent leak of drug solution from the nasal cavity to the esophagus. Administration was conducted by pouring drug solution 25 μl prepared in use using micropipette into the right naris. Blood sample 200 μl was collected from the femoral vein time dependently before and after administration of drug. Blood sample was centrifuged (15,000 rpm/10 min./5°C) and stored at -30 °C before analysis.

QUANTITATIVE METHOD

Blood level was assayed by using human insulin assay reagent (Boehringer Mannheim Co., Germany), an EIA of one step sandwitch method using 2 types of monoclonal antibody.

RESULT

Insulin solution administered group, control 3 shows almost no absorption through the nasal mucosa. Similarly, a solution containing insulin and 0.02 % isosorbide dinitrate (ISDN) (control 6) administered group shows almost no absorption. Significant increase in absorption of insulin from nasal mucosa was observed in the group of the preparation of example 24 added with insulin, 0.5 % LPC and 0.01 % ISDN or 0.01 % nitroglycerin and the group of the preparation of example 25 added with 0.01 % nitroglycerin as compared with that of the prior known conventional preparation of control 4 containing insulin and 0.5 % L-α-lysolecithin (LPC). Area under the curve (AUC ; 0 - 2 hr.) of these administered groups increased about 1.7 fold and 1.5 folds, respectively, as compared with the administered group of the control 4.

EXAMPLE 26

In 1 ml of preparation for transmucosal administration :

| | |
|---|---|
| 1. r-human insulin | 100 units |
| 2. L- α-lysolecithin | 5 mg |
| 3. nitroglycerin | 0.1 mg |
| 4. glycerin | 17.6 mg |

(continued)

| | |
|---|---|
| 5. benzalkonium chloride | 0.1 mg |
| 6. D-mannitol | 10 mg |
| 7. hydrochloric acid/sodium hydroxoide ad libitum for adjusting to pH 6.0 | |
| 8. water for injection | total 1 ml |

The preparation containing the above was prepared. The solution was filtered aseptically (0.22 μm membrane filter) and packed aseptically 3 ml thereof in a vial proper to quantitative mechanical spray for nasal administration to prepare the product. This product contains r-human insulin 100 units/ml and constant spray administration of 10 units of human insulin can be performed by one pushing on adaptor.

EFFECT OF THE INVENTION

A praparation containing physiologically active peptide for transmucosal administration of the present invention shows increasing effect of absorption from nasal mucosa with using minimum amount of absorption promotor without detrimental effect on mucosa.

## Claims

1. A preparation for transmucosal administration comprising a physiologically active peptide, at least admixed with an absorption promotor having absorption promoting actioin for the physiologically active peptide on nasal mucosa or rectal mucosa and a compound having vasodilating activity.

2. The preparation for transmucosal administration according to claim 1 wherein the absorption promotor having absorption promoting action for the physiologically active peptide on nasal mucosa or rectal mucosa has the absorption promoting action with improved absorption rate of above 200 % on nasal mucosa or rectal mucosa as compared with a preparation without absorption promotor when insulin is used as the physiologically active peptide.

3. The preparation for transmucosal administration according to claim 1 wherein the absorption promotor is one or more numbers thereof selected from the group consisting of salt of bile acid, salt of fusidic acid, salt of glycyrrhizic acid, salt of O-acyl-L-carnitine, phospholipid, non-ionic surface active agent, cyclodextrin, higher fatty acid, 1-alkyl-2-pyrrolidone derivative, 1-dodecylazacycloheptane-2-one, bacitracin, sodium azulenesulfonate and azacycloalkane derivativeof the formula 〔1〕

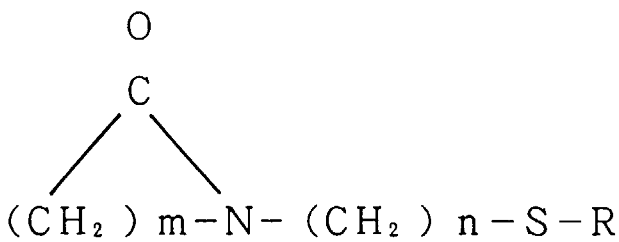

〔1〕

wherein R is an alkyl, m is an integer of 2 - 4 and n is an integer of 1 - 15, provided that R is an alkyl with a carbon number of 5 - 11 in case where n is 1 - 3.

4. The preparation for transmucosal administration according to claim 3 wherein salt of bile acid is one or more numbers thereof selected from the group consisting of sodium taurocholate, sodium glycocholate and sodium deoxycholate.

5. The preparation for transmucosal administration according to claim 3 wherein salt of fusidic acid is one or more numbers thereof selected from the group consisting of sodium fusidic acid and tauro-24, 25-dihydrofusidic acid.

6. The preparation for transmucosal administration according to claim 3 wherein salt of glycyrrhizic acid is one or

more numbers thereof selected from the group consisting of salt of glycyrrhizic acid and disodium 3-succinyloxyglycyrrhizic acid (carbenixolon).

7. The preparation for transmucosal administration according to claim 3 wherein salt of O-acyl-L-carnitine is O-acyl-L-carnitine having $C_{8-18}$ acyl.

8. The preparation for transmucosal administration according to claim 3 wherein salt of O-acyl-L-carnitine is one or more numbers thereof selected from the group consisting of salt of O-octanoyl-L-carnitine, salt of O-lauroyl-L-carnitine and salt of O-palmitoyl-L-carnitine.

9. The preparation for transmucosal administration according to claim 3 wherein phospholipid is one or more numbers thereof selected from the group consisting of phosphatidylcholine (lecithin), lisophosphatidylcholine (lysolecithin) and lysophosphatidylglycerol.

10. The preparation for transmucosal administration according to claim 3 wherein non-ionic surface active agent is one or more numbers thereof selected from the group consisting of polyoxyalkylene higher alcohol ether, polyoxyalkylene alkylphenol and sucrose fatty acid ester.

11. The preparation for transmucosal administration according to claim 3 wherein non-ionic surface active agent is one or more numbers thereof selected from the group consisting of polyoxyalkylene lauryl and polyoxyalkylene (24) cholesteryl ether.

12. The preparation for transmucosal administration according to claim 3 wherein cyclodextrin is one or more numbers thereof selected from the group consisting of $\alpha$-cyclodextrin, $\beta$-cyclodextrin, $\gamma$-cyclodextrin and dimethyl- $\beta$-cyclodextrin.

13. The preparation for transmucosal administration according to claim 3 wherein higher fatty acid is higher fatty acid of $C_{16-20}$ .

14. The preparation for transmucosal administration according to claim 13 wherein higher fatty acid of $C_{16-20}$ is one or more numbers of $C_{18}$ higher fatty acid selected from the group consisting of oleic acid, linoleic acid and linolenic acid.

15. The preparation for transmucosal administration according to claim 3 wherein 1-alkyl-2-pyrrolidone derivative is one or more numbers thereof selected from the group consisting of $C_{4-12}$ alkyl.

16. The preparation for transmucosal administration according to claim 15 wherein alkyl is one or more numbers thereof selected from the group consisting of butyl, pentyl, hexyl, heptyl, octyl, nonyl, decyl, undecyl and dodecyl.

17. The preparation for transmucosal administration according to claim 3 wherein azacycloalkane derivative of the formul 〔1〕 is azacycloalkane derivative in which R is $C_{10}$ alkyl, m is 3 and n is 2, i.e. 1-[2-(decylthio) ethyl] azacyclopentane-2-one.

18. The preparation for transmucosal administration according to claim 1 wherein the absorption promotor is admixed with 0.01 - 5 weight % of the said preparation.

19. The preparation for transmucosal administration according to claim 1 wherein the compound having vasodilating activity is one or more numbers thereof selected from the group consisting of calcium channel blocker of molecular weight 200 - 700, prostaglandin E1, isosorbide dinitrate and nitroglycerin.

20. The preparation for transmucosal administration according to claim 19 wherein calcium channel blocker is diltiazem hydrochloride, verapamil hydrochloride, bepridil hydrochloride. nifedipine hydrochloride, nicardipine hydrochloride and fasudil hydrochloride.

21. The preparation for transmucosal administration according to claim 1 wherein the compound having vasodilating activity is admixed with below 1/2 of minimum usual dose as an effective component of the said compound in the preparation for transmucosal administration.

**22.** The preparation for transmucosal administration according to claim 1 wherein molecular weight of the physiologically active peptide is 300 - 10,000.

**23.** The preparation for transmucosal administration according to claim 1 wherein the physiologically active peptide is selected from the group consisting of insuline, calcitonin, human PTH (1 - 34), calcitonin gene related peptide (CGRP), angiotensin II, vasopressin. desmopressin acetate, buserelin acetate, goserelin acetate, nafarelin acetate, leuprorelin acetate, somatostatin, glucagon, oxytocin, secretin, LH - RH, ACTH, TRH, TSH, ANP or derivatives containing synthetic or semisynthetic compound thereof.

**24.** The preparation for transmucosal administration according to claim 23 wherein calcitonin is a compound selected from the group consisting eel calcitonin, salmon calcitonin, porcine carcitonin, human calcitonin and chicken carcitonin.

**25.** The preparation for transmucosal administration according to claim 24 wherein eel calcitonin is ASU $^{1-7}$ eel calcitonin (elcatonin).

**26.** The preparation for transmucosal administration according to claim 23 wherein insulin is a compound selected from the group consisting human insulin, porcine insulin and bovine insulin.

**27.** The preparation for transmucosal administration according to claim 1 wherein the preparation for transmucosal administration is at least one of the preparation for administration in nasal mucosa, oral mucosa, pulmonary mucosa, rectal mucosa, vaginal mucosa or ocular mucosa.

FIG. 1

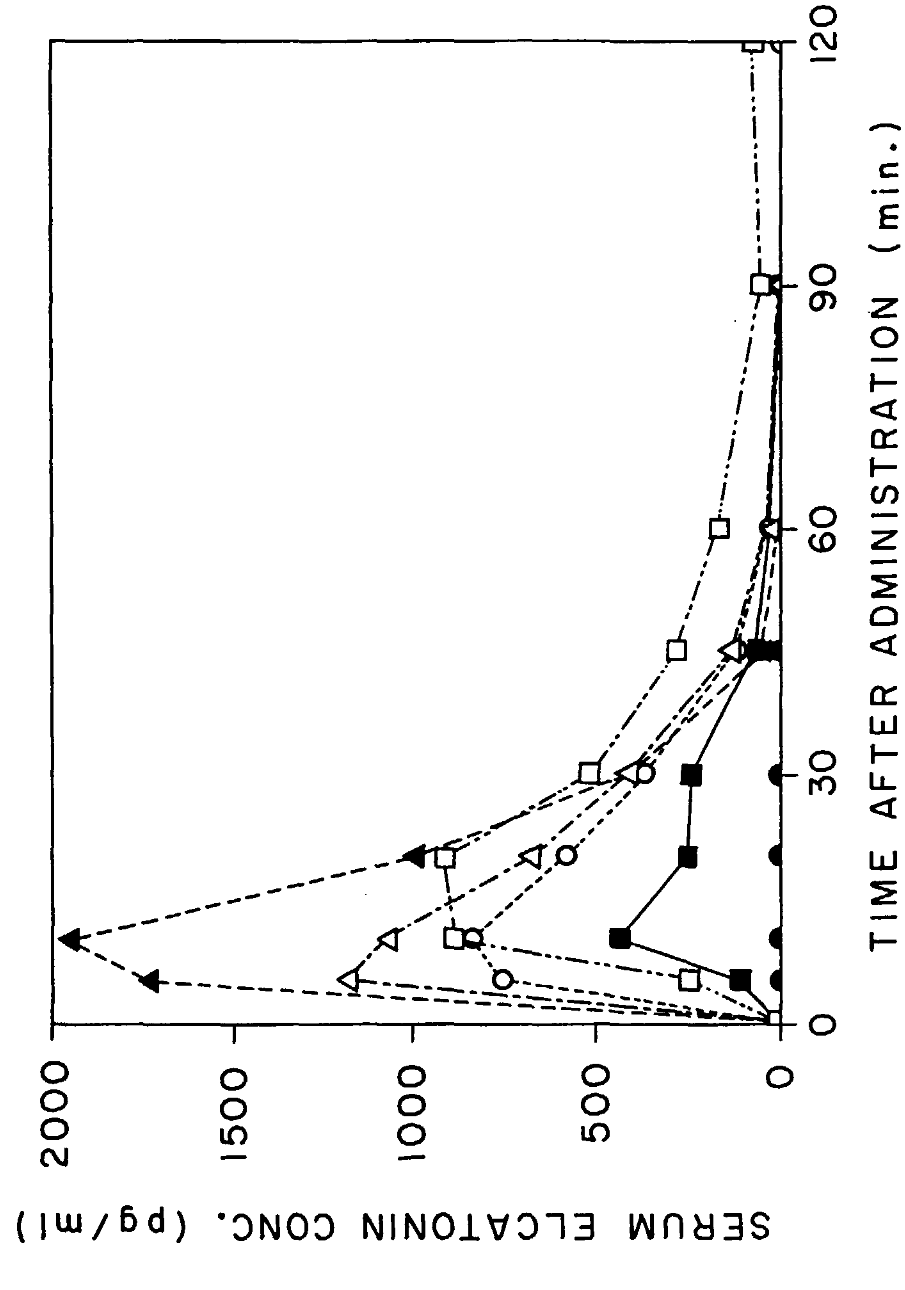
SERUM ELCATONIN CONC. (pg/ml)
2000
1500
1000
500
0
0
30
60
90
120
TIME AFTER ADMINISTRATION (min.)

FIG. 2

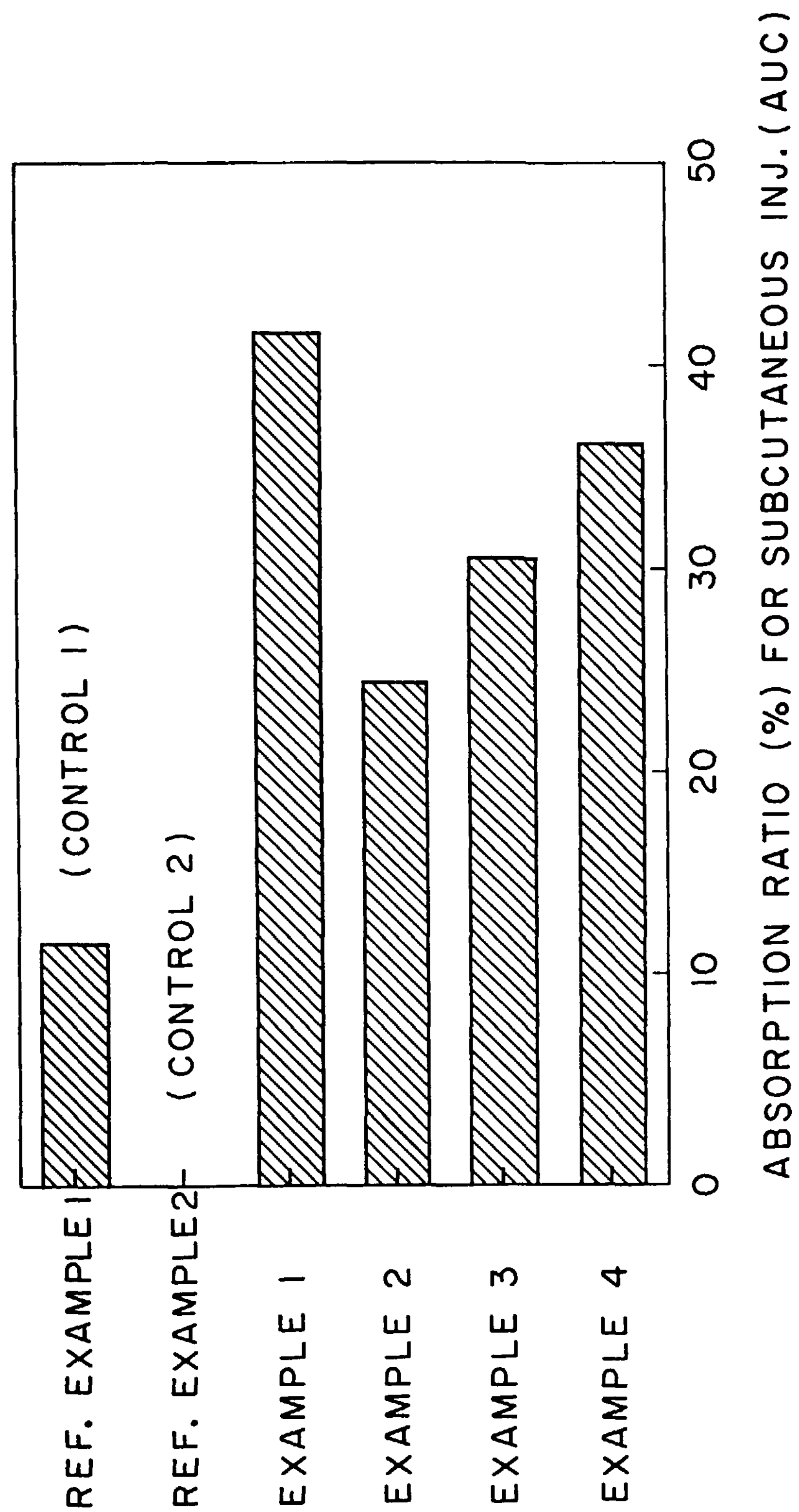
REF. EXAMPLE 1
(CONTROL 1)
REF. EXAMPLE 2
(CONTROL 2)
EXAMPLE 1
EXAMPLE 2
EXAMPLE 3
EXAMPLE 4
0
10
20
30
40
50
ABSORPTION RATIO (%) FOR SUBCUTANEOUS INJ. (AUC)

FIG. 3

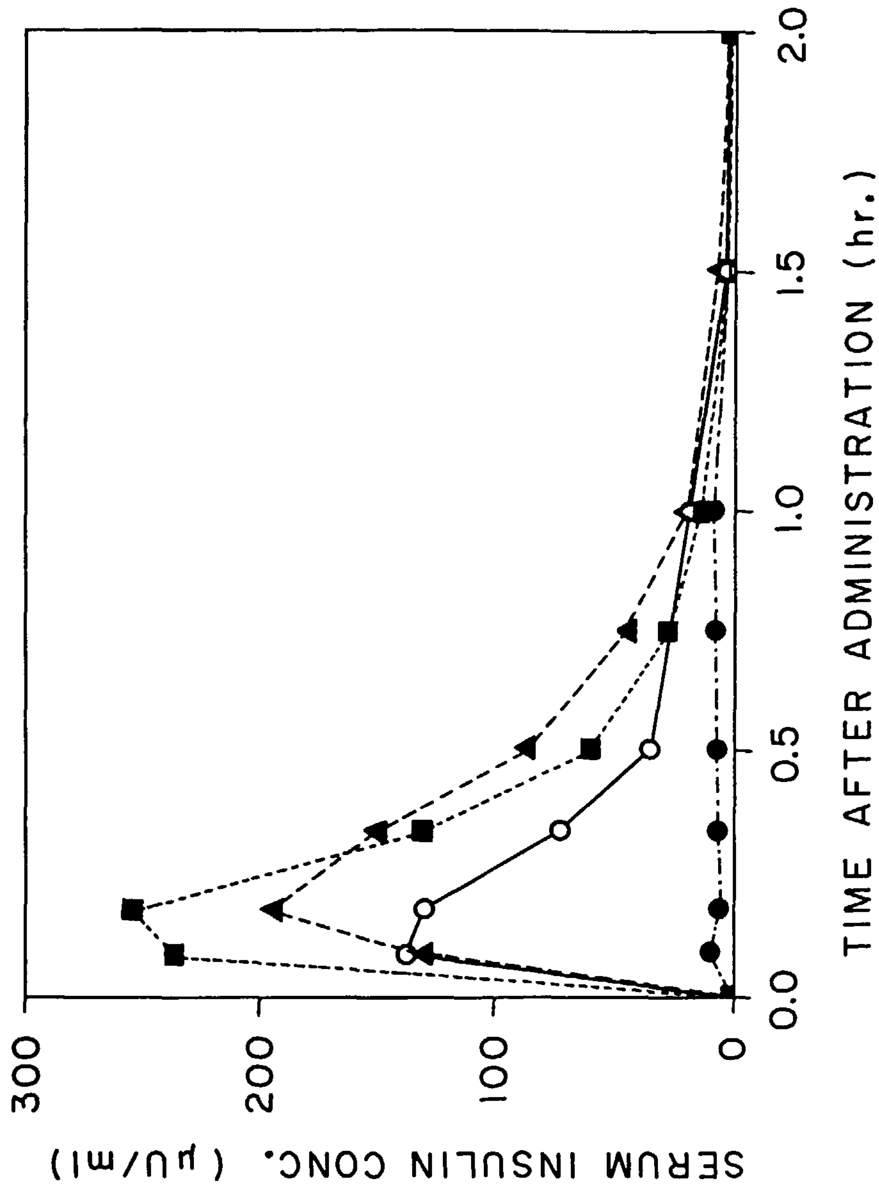
SERUM INSULIN CONC. ( μU/ml)
300
200
100
0
0.0
0.5
1.0
1.5
2.0
TIME AFTER ADMINISTRATION (hr.)

FIG. 4

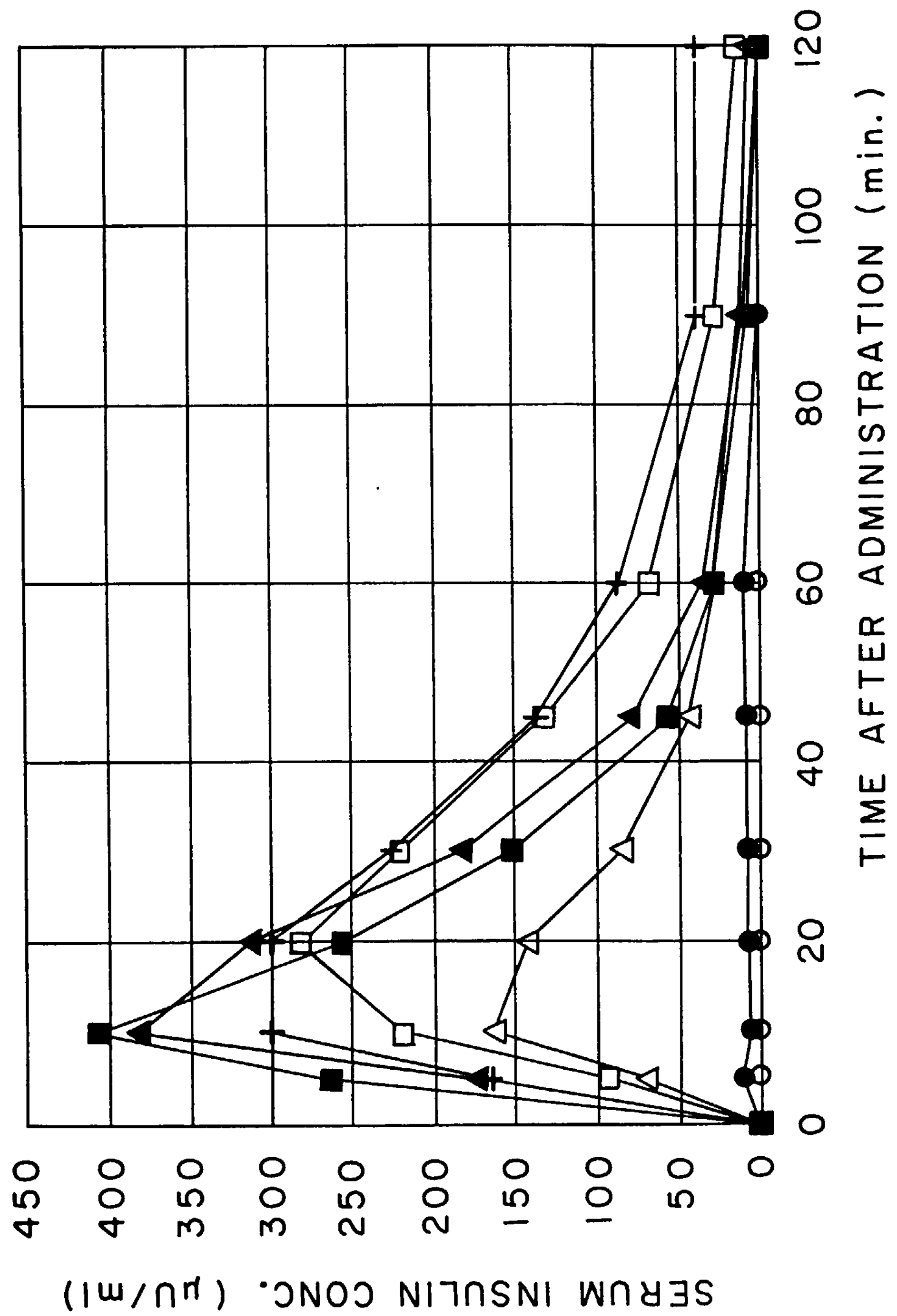
SERUM INSULIN CONC. (μU/ml)
0
50
100
150
200
250
300
350
400
450
TIME AFTER ADMINISTRATION (min.)
0
20
40
60
80
100
120

FIG. 5

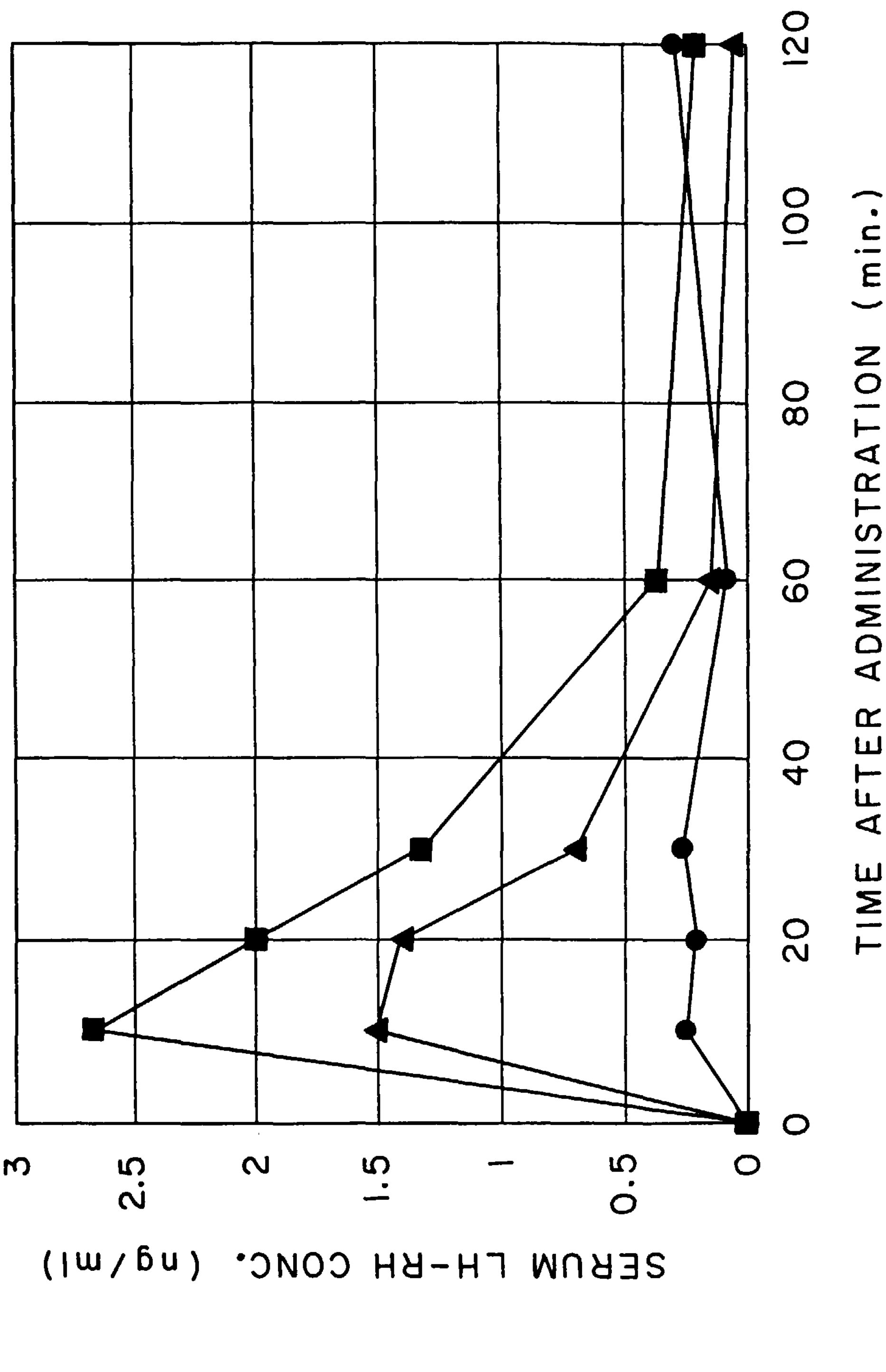
SERUM LH-RH CONC. (ng/ml)
0
0.5
1
1.5
2
2.5
3
TIME AFTER ADMINISTRATION (min.)
0
20
40
60
80
100
120

**INTERNATIONAL SEARCH REPORT**

International application No.
PCT/JP96/02277

**A. CLASSIFICATION OF SUBJECT MATTER**

Int. $Cl^6$ A61K38/00, A61K9/00

According to International Patent Classification (IPC) or to both national classification and IPC

**B. FIELDS SEARCHED**

Minimum documentation searched (classification system followed by classification symbols)

Int. $Cl^6$ A61K38/00, A61K9/00

Documentation searched other than minimum documentation to the extent that such documents are included in the fields searched

Electronic data base consulted during the international search (name of data base and, where practicable, search terms used)

Database WPIL on DIALOG

**C. DOCUMENTS CONSIDERED TO BE RELEVANT**

| Category* | Citation of document, with indication, where appropriate, of the relevant passages | Relevant to claim No. |
|---|---|---|
| A | JP, 58-189118, A (Takeda Chemical Ind. K.K.), November 4, 1983 (04. 11. 83) & US, 4659696, A & EP, 94157, A | 1-3, 12, 18, 22-27 |
| A | JP, 3-505462, A (Ney U. M.), November 28, 1991 (28. 11. 91) & EP, 433402, A & WO, 90/11769 | 1, 19-23 |
| A | EP, 566135, A (Takeda Chemical Ind. K.K.), October 20, 1993 (20. 10. 93) & JP, 6-9424, A & US, 5482706, A | 1, 2, 19 - 27 |
| A | JP, 1-501550, A (Novo-Nordisk AS.), June 1, 1989 (01. 06. 89) & EP, 272097, A & WO, 88-4556 & US, 4179079, A | 1-3, 9, 18, 22, 23, 27 |
| A | JP, 59-130820, A (Armour Pharm Co.), July 27, 1984 (27. 07. 84) & EP, 115627, A | 1, 3, 4, 10, 18, 22 - 27 |
| A | JP, 4-230223, A (Sandoz AG.), | 1, 3, 4, |

[X] Further documents are listed in the continuation of Box C. [ ] See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| October 30, 1996 (30. 10. 96) | November 12, 1996 (12. 11. 96) |
| Name and mailing address of the ISA/ Japanese Patent Office | Authorized officer |
| Facsimile No. | Telephone No. |

Form PCT/ISA/210 (second sheet) (July 1992)

**INTERNATIONAL SEARCH REPORT**

International application No. PCT/JP96/02277

C (Continuation). DOCUMENTS CONSIDERED TO BE RELEVANT

| Category* | Citation of document, with indication, where appropriate, of the relevant passages | Relevant to claim No. |
|---|---|---|
| | August 19, 1992 (19. 08. 92)<br>& EP, 462071, A & FR, 2663227, A | 18, 22,<br>23, 27 |

Form PCT/ISA/210 (continuation of second sheet) (July 1992)